# EUROPÄISCHE PATENTSCHRIFT

(11) **EP 2 627 388 B1**
(45) Veröffentlichungstag und Bekanntmachung des Hinweises auf die Patenterteilung: **12.07.2017**
(21) Anmeldenummer: 11804934.5
(22) Anmeldetag: 28.09.2011
(51) Int. Cl.: A61M 16/04, A61M 16/10

(54) **KÜNSTLICHE NASE MIT SPRECHVENTIL**
ARTIFICIAL NOSE COMPRISING A SPEECH VALVE
NEZ ARTIFICIEL COMPORTANT UNE VALVE PHONATOIRE

(30) Priorität: 14.10.2010 DE 102010048317
(43) Veröffentlichungstag der Anmeldung: 21.08.2013
(73) Patentinhaber: Primed Halberstadt Medizintechnik GmbH, 28820 Halberstadt (DE)
(72) Erfinder: LEIBITZKI, Harry, 38889 Blankenburg (DE); SÜSS, Steffen, 38820 Halberstadt (DE)
(74) Vertreter: Donath, Dirk
(86) Internationale Anmeldenummer: PCT/DE2011/001807
(87) Internationale Veröffentlichungsnummer: WO 2012/048681

(56) Entgegenhaltungen:
- WO-A1-99/29268
- WO-A1-2010/060983
- WO-A2-2008/132222
- US-A- 2 996 071
- US-A- 5 738 095

## Beschreibung

Die vorliegende Erfindung betrifft eine künstliche Nase mit Sprechventil in Form einer Vorrichtung zum Einsetzen in eine Tracheostoma-Prothese oder ein Tracheostoma- Pflaster.

Tracheostoma- Prothesen (auch Trachealkanülen oder Tracheostoma-Tuben genannt) zur Behandlung kehlkopfloser (laryngektomierter) Patienten mit eröffneter Kehle (s.g. Tracheostoma) sind seit Jahrzehnten bekannt. Diese Prothesen gibt es als Ausführungen mit und ohne Sprechventil, wobei in den letzten Jahren die verschiedenen Ausführungsformen mit Sprechventil immer mehr an Bedeutung bei der Rehabilitation der laryngektomierten Patienten gewonnen haben, da durch sie Sprecherfolge bei den Patienten ermöglicht werden.

Die Tracheostoma- Tuben mit Sprechventil bestehen zumeist aus einer Trachealkanüle mit Kanülenschild und einem Ventilaufnahmeteil zur Halterung eines äußeren Sprechventils, wobei ein inneres Sprechventil der Trachealkanüle in einer Röhre zwischen Speiseröhre und Luftröhre gelagert ist, so dass bei blockiertem Tracheostoma (bei verschlossenem äußeren Sprechventil) Luft durch das innere Sprechventil in Richtung Mundraum entweichen kann und dabei im oberen Bereich der Speiseröhre Schwingungen zum Sprechen erzeugt werden. Das Blockieren des Tracheostoma erfolgt dabei manuell durch Verschließen des äußeren Sprechventils an der Außenseite der Trachealkanüle.

Auf Grund einer Kehlkopfentfernung und des anschließenden Einsatzes einer Tracheostoma- Prothese wird die Verbindung zwischen Nase und Lunge des laryngektomierten Patienten unterbrochen, so dass die natürliche Funktion der Nase (Erwärmung, Befeuchtung und Filterung der Atemluft, sowie Aufbau eines gewissen Atemwiderstandes) nicht mehr gewährleistet sind.

Gemäß dem Stand der Technik sind Feuchte- und Wärme-Austauschvorrichtungen (auch als künstliche Nasen oder HME bezeichnet) zum Aufsetzen auf Trachealkanülen oder Tracheostoma-Tuben bekannt.
Die Feuchte- und Wärme- Austauschvorrichtungen entziehen der durch den Patienten ausgeatmeten Luft die Feuchtigkeit und Wärme und geben diese wieder an die Einatmenluft ab, wobei die Atemluft gleichzeitig gefiltert wird, so dass das Eindringen von Partikeln in die Atemwege des Patienten verhindert und durch den Filter ein gewisser Atemwiderstand aufgebaut wird.

DE 694 05 587 T2 offenbart eine Vorrichtung zum Einsetzen in ein Tracheostoma mit einem Filtergehäuse zur Aufnahme eines Feuchtigkeit und Wärme tauschenden Filters, wobei das Filtergehäuse eine erste Öffnung zur Verbindung mit dem Stoma des Patienten und wenigstens eine zweite Öffnung an der gegenüberliegenden Seite des Filters in einer Strömungsrichtung der Atmungsluft aufweist. Dabei ist ein Ventilelement in der zweiten Öffnung des Filtergehäuses zum Schließen der zweiten Öffnung vorgesehen, wobei das Ventilelement geeignet ist, mittels eines Fingers manuell geschlossen und durch federndes Zurückkehren geöffnet zu werden.

WO 2010/060983 A1 beschreibt einen Atemschutz in einem Tracheostoma. WO 2008/132222 A2 bescheibt einen Atemschutz für den Einsatz in einem Stoma einer laryngektomierten oder tracheotomierten Person.

WO 99/29268 A1 beschreibt eine Sprachventilanordnung zur Verbindung mit dem Tracheostoma eines Kehlkopflosers.

US 5 738 095 A beschreibt ein Tracheostoma-Gerät. Der Erfindung liegt die Aufgabe zugrunde, eine Vorrichtung zum Einsetzen in ein Tracheostoma anzugeben, die ein Sprechen des laryngektomierten Patienten vermittels ein- und ausgeführter Atemluft bei gleichzeitiger Erwärmung, Befeuchtung und Filterung der Atemluft sowie den Aufbau eines gewissen Atemwiderstandes ermöglicht, und gleichzeitig ein Einfließen von Speichel in die Luftröhre verhindert, so dass die Atemweg des Patienten geschont und ein Hustenreiz des Patienten in Folge des Sprechens verhindert wird. Insbesondere sollen bei der bestimmungsgemäßen Verwendung der Vorrichtung größere Partikel in einer Art Selbstreinigung von dem verwendeten Filter entfernt werden.
Gelöst wird diese Aufgabe durch eine Vorrichtung zum Einsetzen in ein Tracheostoma gemäß dem ersten Patentanspruch. Vorteilhafte Ausgestaltungen der Erfindung sind in den nachgeordneten Ansprüchen angegeben.

Das Wesen der Erfindung besteht in der Bereitstellung einer neuartigen Vorrichtung zum Einsetzen in eine Tracheostoma- Prothese oder ein Tracheostoma- Pflaster, welches ein Gehäuse und ein Ventilelement umfasst, wobei das Gehäuse eine erste Öffnung zur Verbindung mit dem Stoma eines Patienten sowie wenigstens eine zweite Öffnung an der gegenüberliegenden Seite aufweist und das Ventilelement in der zweiten Öffnung des Gehäuses zum Schließen der zweiten Öffnung vorgesehen ist, und bei dem das Ventilelement geeignet ist, mittels eines Fingers manuell geschlossen und durch federndes Zurückkehren geöffnet zu werden, wobei das Gehäuse das Ventilelement umschließt, das Ventilelement einen Feuchtigkeit und Wärme tauschenden Filter aufnimmt und ein zylindrisches Federelement zwischen dem Gehäuse und dem Ventilelement angeordnet ist.
Dabei weist das Gehäuse gemäß der Erfindung einen oberen und einen unteren Führungsring auf, zwischen denen sich Führungsstege mit laterale Ausnehmungen (Öffnungen) für den Durchtritt von Atemluft befinden.

Das erfindungsgemäße Ventilelement verfügt über einen Deckel sowie eine Wand mit lateralen Ausnehmungen, wobei diese Ausnehmungen in Position und Form mit den Öffnungen des Gehäuses korrespondieren, so dass die Atemluft bei bestimmungsgemäßer Verwendung der Vorrichtung durch diese hindurch treten kann. An der Wand unter dem Deckel sind mindestens zwei Führungsnasen vorgesehen, die in die Ausnehmungen der Führungsstege des Gehäuses greifen, so dass die Führungsnasen in den Führungsstegen zwischen zwei Endpunkten bewegbar sind.

Der Filter ist zwischen dem Deckel und der Wand des Ventilelements angeordnet, wobei das Federelement unterhalb des Filters zwischen dem Ventilelement und dem Gehäuse gehaltert wird.

Die erfindungsgemäße Vorrichtung ist als Einwegartikel aus Kunststoffen gefertigt und leicht mit bekannten Trachealkanülen, Tracheostoma- Tuben oder Tracheostoma- Pflastern koppelbar, indem die äußere Wand des Gehäuse eine üblichen Durchmesser aufweist und an der, dem Stroma zugewandten Seite mit eine Wulst versehen ist, so dass ein Aufstecken und Arritieren der Vorrichtung an den Trachealkanülen, Tracheostoma- Tuben oder Tracheostoma- Pflastern ermöglicht ist.

Der Vorteil der erfindungsgemäßen Vorrichtung besteht darin, dass der Filter aus dem Gehäuse bis zum äußeren Gehäuserand heraus ragt, so dass grobe Partikel der eingeführten Atemluft in den Ausnehmungen zurückgehalten und beim Ausatmen abgeführt werden können, was einem schnellen Verstopfen der Vorrichtung entgegen wirkt.

Darüber hinaus hat die erfindungsgemäße Vorrichtung den Vorteil, dass sie einen sehr flachen Aufbau besitzt und dass sie bei bestimmungsgemäßer Verwendung auf Grund ihrer lateralen Ausnehmungen für den Luftauslass gegenüber dem Stand der Technik bessere Atemkennwerte aufweist.

Die Erfindung wird nachstehend an Hand des Ausführungsbeispiels und der Figuren näher erläutert. Dabei zeigt:
- Fig. 1a:: eine schematische 3-D-Darstellung einer Ausführungsform einer erfindungsgemäßen Vorrichtung in einer ersten Blickrichtung,
- Fig. 1b:: eine schematische 3-D-Darstellung der Ausführungsform der erfindungsgemäßen Vorrichtung gemäß Fig. 1 in einer zweiten Blickrichtung,
- Fig. 2a:: eine schematische 3-D-Darstellung des Gehäuses gemäß Fig. 1a und 1b,
- Fig. 2b:: eine schematische 3-D-Darstellung des Ventilelements gemäß Fig. 1 und 1b,
- Fig. 2c:: eine schematische 3-D-Darstellung des Federelements gemäß Fig. 1a und 1b,
- Fig. 3a:: eine Draufsicht auf die Vorrichtung gemäß Fig. 1 und
- Fig. 3b:: eine Schnittdarstellung der Vorrichtung entlang der Ebene - A-A- gemäß Fig. 3a mit gedrücktem Federelement (entspricht dem geschlossenen Zustand der Vorrichtung).

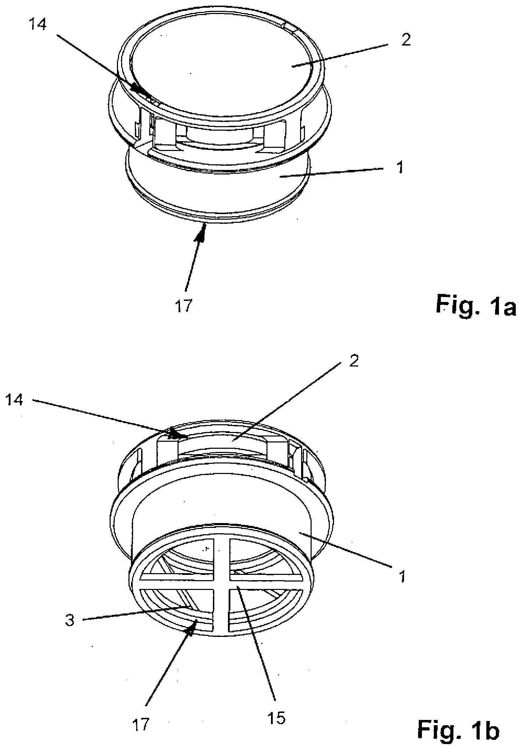

Fig. 1a und 1b zeigen eine Vorrichtung zum Einsetzen in eine Tracheostoma- Prothese oder ein Tracheostoma- Pflaster umfassend ein zylindrisches Gehäuse (1) und ein zylindrisches Ventilelement (2), wobei das Gehäuse (1) eine erste Öffnung (17) zur Verbindung mit dem Stoma des Patienten sowie wenigstens eine zweite Öffnung (14) an der gegenüberliegenden Seite aufweist und das Ventilelement (2) in der zweiten Öffnung (14) des Gehäuses (1) zum Schließen der zweiten Öffnung (14) vorgesehen ist, wobei das Ventilelement (2) geeignet ist, mittels eines Fingers manuell geschlossen und durch federndes Zurückkehren geöffnet zu werden.

Das Gehäuse (1) umschließt dabei das Ventilelement (2).

Das Ventilelement (2) nimmt einen Feuchtigkeit und Wärme tauschenden Filter auf (in der Figur nicht dargestellt), wobei ein zylindrisches Federelement (3) unterhalb des Filters zwischen dem Gehäuse (1) und dem Ventilelement (2) angeordnet ist.

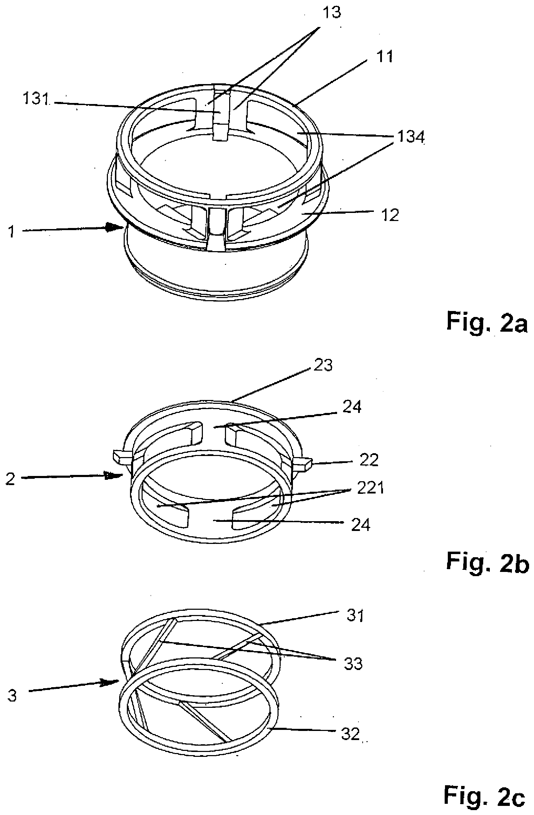

Das Gehäuse (1) weist, wie in Fig. 2a dargestellt, einen oberen Führungsring (11) und einen unteren Führungsring (12) auf, zwischen denen sich paarweise Führungsstege (13) mit zwischengelegenen Ausnehmungen (131) befinden, wobei zwischen den paarweisen Führungsstegen (13) lateralen Öffnungen (134) ausgebildet sind, so dass Luft durch diese ein und ausführbar ist.

Das Ventilelement (2) weist, wie in Fig. 2b dargestellt, einen Deckel (23) sowie eine Wand (24) mit lateralen Ausnehmungen (221) auf, wobei diese Ausnehmungen (221) in Position und Form mit den lateralen Öffnungen (134) des Gehäuses (1) korrespondieren, und an der Wand (24) unter dem Deckel (23) mindestens zwei Führungsnasen (22) vorgesehen sind, die in die Ausnehmungen (131) der Führungsstege (13) des Gehäuses (1) greifen. Die Führungsnasen (22) sind in den Führungsstegen (13) zwischen zwei Endpunkten bewegbar.

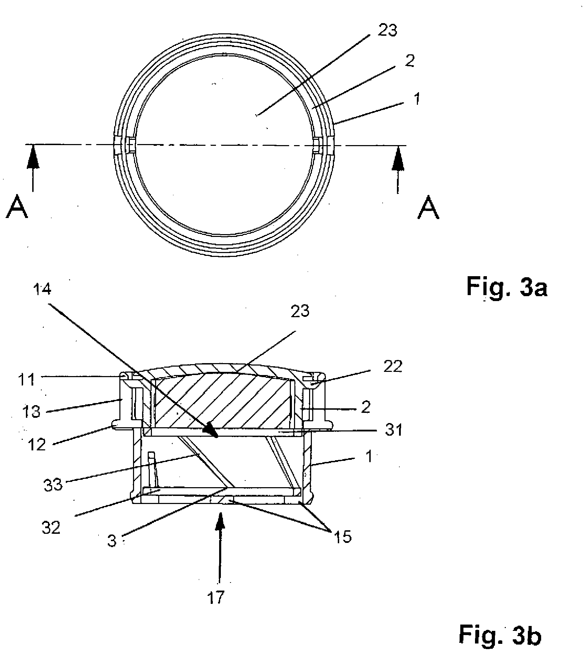

Der nicht in der Zeichnung dargestellte Filter wird vom Ventilelement (2) umschlossen. Dabei ist er zwischen dem Deckel (23) und der Wand (24) des Ventilelements (2) positioniert, wobei das in Fig. 3c dargestellte Federelement (3) unterhalb des Filters zwischen Gehäuse (1) und Ventilelement (2) angeordnet ist.

Das Gehäuse (1) weist auf der gegenüberliegenden Seite der Führungsringe (11; 12) radiale Arme (15) auf.

Das in Fig. 3c dargestellte Federelement (3) besteht aus einem oberen Federring (31) und einem unteren Federring (32), wobei die Federringe (31 und 32) durch elastische Federarme (33) miteinander verbunden sind.

Der untere Rand der Wand (24) des Ventilelements (2) und die radialen Arme (15) des Gehäuses (1) haltern die Federringe (31 und 32).

Die Führungsnasen (22) sind in den Ausnehmungen (131) der Führungsstege (13) zwischen zwei Endlagen bewegbar.

Die erfindungsgemäße Vorrichtung ist als Einwegprodukt in geeigneter Weise aus Kunststoff gefertigt.

So bestehen das Gehäuse (1) und das Ventilelement (2) bspw. aus Polyethylen (PE) oder Polypropylen (PP).

Das Federelement (3) ist bspw. aus Polypropylen (PP) oder Polyethylen (PE) gefertigt. Alternativ dazu kann es jedoch auch als Spiralfeder aus medizinisch geeignetem Metall, wie bspw. Federstahl bestehen.

Der Filter besteht bspw. aus geschäumtem Polyurethan.

Wenn die erfindungsgemäße Vorrichtung bestimmungsgemäß verwendet wird, ist der Filter in dem Ventilelement (2) so angeordnet, dass das Federelement (3) unterhalb des Filters positioniert ist und sich der obere Federring (31) des Federelements (3) am unteren Ende der Wand (24) des Ventilelements (2) und sich der untere Federring (32) des Federelements (3) an den Armen (15) des Gehäuses (1) abstützt. Vermittels der beweglichen Federarme (33) ist das Federelement spannbar (gedrückte Stellung des Federelements) bzw. entspannbar (nicht gedrückte Stellung des Federelements), was zu einem Schließen bzw. Öffnen der zweiten Öffnung (14) im Gehäuse (1) vermittels des Deckels (23) des Ventilelements (2) führt.

Zum Sprechen wird der Deckel (23) von einem Finger des Patienten leicht gedrückt, so dass der zuvor stehende Schließvorgang zwischen zweiter Öffnung (14) und Deckel (23) generiert wird (siehe Fig. 3b). Nach dem Sprechen wird der Finger entfernt, so dass der Deckel (23), durch die Kraft der Feder bewegt, die zweite Öffnung (14) wieder frei gibt und die Atemluft durch die lateralen Ausnehmungen (221) des Ventilelements (2) und die lateralen Öffnungen (14) des Gehäuses (1) gelangen kann.
Der in dem Ventilelement (2) positionierte Filter hält dabei beim Einatmen des Patienten Partikel [insbesondere im Bereich der Öffnungen (14)] zurück, die beim Ausatmen aus dem Filter ausgeblasen werden, so dass ein Verstopfen des Filters durch diese Partikel verhindert wird.

Alle in der Beschreibung, den Ausführungsbeispielen und den nachfolgenden Ansprüchen dargestellten Merkmale können sowohl einzeln als auch in beliebiger Kombination miteinander erfindungswesentlich sein.

### Bezugszeichenliste

- 1: - Gehäuse
- 11: - oberer Führungsring
- 12: - unterer Führungsring
- 13: - Führungssteg
- 131: - Ausnehmungen
- 134: - laterale Öffnung
- 14: - zweite Öffnung
- 15: - Arm
- 17: - erste Öffnung
- 2: - Ventilelement
- 221: - Ausnehmungen
- 22: - Führungsnase
- 23: - Deckel
- 24: - Wand
- 3: - Federelement
- 31: - oberer Federring
- 32: - unterer Federring
- 33: - Federarme

## Patentansprüche

1. Vorrichtung zum Einsetzen in eine Tracheostoma- Prothese oder ein Tracheostoma- Pflaster umfassend ein zylindrisches Gehäuse (1) und ein zylindrisches Ventilelement (2), wobei
• das Gehäuse (1) eine erste Öffnung (17) zur Verbindung mit dem Stoma des Patienten sowie wenigstens eine zweite Öffnung (14) an der gegenüberliegenden Seite aufweist und das Ventilelement (2) in der zweiten Öffnung (14) des Gehäuses (1) zum Schließen der zweiten Öffnung (14) vorgesehen ist, wobei das Ventilelement (2) geeignet ist, mittels eines Fingers manuell geschlossen und durch federndes Zurückkehren geöffnet zu werden,
• das Gehäuse (1) das Ventilelement (2) umschließt und das Ventilelement (2) einen Feuchtigkeit und Wärme tauschenden Filter aufnimmt, wobei ein zylindrisches Federelement (3) zwischen dem Gehäuse (1) und dem Ventilelement (2) angeordnet ist,
• das Gehäuse (1) einen oberen Führungsring (11) und einen unteren Führungsring (12) aufweist, zwischen denen sich paarweise Führungsstege (13) mit zwischengelegenen Ausnehmungen (131) befinden, wobei zwischen den paarweisen Führungsstegen (13) laterale Öffnungen (134) ausgebildet sind,
• das Ventilelement (2) einen Deckel (23) sowie eine Wand (24) mit lateralen Ausnehmungen (221) aufweist, wobei diese Ausnehmungen (221) in Position und Form mit den lateralen Öffnungen (134) des Gehäuses (1) korrespondieren und an der Wand (24) unter dem Deckel (23) mindestens zwei Führungsnasen (22) vorgesehen sind, die in die Ausnehmungen (131) der Führungsstege (13) des Gehäuses (1) greifen und
• der Filter zwischen dem Deckel (23) und der Wand (24) des Ventilelements (2) positioniert ist, wobei das Federelement (3) unterhalb des Filters zwischen Gehäuse (1) und Ventilelement (2) angeordnet ist,
**dadurch gekennzeichnet, dass** das Federelement (3) aus einem oberen Federring (31) und einem unteren Federring (32) besteht, wobei die Federringe (31 und 32) durch elastische Federarme (33) miteinander verbunden sind.

2. Vorrichtung gemäß Anspruch 1, **dadurch gekennzeichnet, dass** das Gehäuse (1) auf der gegenüberliegenden Seite der Führungsringe (11; 12) radiale Arme (15) aufweist.

3. Vorrichtung gemäß Anspruch 1, **dadurch gekennzeichnet, dass** der untere Rand der Wand (24) des Ventilelements (2) und die radialen Arme (15) des Gehäuses (1) die Federringe (31 und 32) haltern und die Führungsnasen (22) in den Ausnehmungen (131) der Führungsstege (13) zwischen zwei Endlagen bewegbar sind.

4. Vorrichtung gemäß Anspruch 1, 2 oder 3, **dadurch gekennzeichnet, dass** das Gehäuse (1) und das Ventilelement (2) aus Polyethylen oder Polypropylen bestehen.

5. Vorrichtung gemäß Anspruch 1, 2, 3 oder 4, **dadurch gekennzeichnet, dass** das Federelement (3) aus Polyethylen oder Polypropylen oder Federstahl besteht.

6. Vorrichtung gemäß Anspruch 1, 2, 3, 4 oder 5, **dadurch gekennzeichnet, dass** der Filter aus geschäumten Polyurethan besteht.

## Claims

1. A device for insertion into a tracheostoma prosthesis or a thracheostoma plaster comprising a cylindrical housing (1) and a cylindrical valve element (2), wherein
• the housing (1) is provided with a first opening (17) for the connection to the patient's stoma and with at least one second opening (14) on the opposite side, and the valve element (2) is provided in the second opening (14) of the housing (1) to close the second opening (14), wherein the valve element (2) is suited to be closed manually by means of a finger and to be opened by elastic return,
• the housing (1) encloses the valve element (2) and the valve element (2) receives a heat and moisture exchanging filter, wherein a cylindrical spring element (3) is arranged between the housing (1) and the valve element (2),
• the housing (1) has an upper guide ring (11) and a lower guide ring (12) and guide rods (13) are arranged in pairs between them and have recesses in between (131), wherein lateral openings (134) are provided between the pairwise-arranged guide rods (13),
• the valve element (2) is provided with a cover (23) and a wall (24) with lateral recesses (221), wherein the position and shape of these recesses (221) correspond to the lateral openings (134) of the housing (1), and at the wall (24) under the cover (23) at least two guide projections (22) are arranged that engage in the recesses (131) of the guide rods (13) of the housing (1), and
• the filter is positioned between the cover (23) and the wall (24) of the valve element (2), wherein the spring element (3) is arranged below the filter between the housing (1) and the valve element (2), **characterized in that** the spring element consists of an upper spring ring (31) and a lower spring ring (32), wherein the spring rings (31 and 32) are connected to each other by elastic spring arms (33).

2. The device according to claim 1, **characterized in that** the housing (1) is provided with radial arms (15) on the opposite side of the guide rings (11; 12).

3. The device according to claim 1, **characterized in that** the lower edge of the wall (24) of the valve element (2) and the radial arms (15) of the housing (1) support the spring rings (31 and 32), and the guide projections (22) are movable between two end positions in the recesses (131) of the guide rods (13).

4. The device according to claim 1, 2 or 3, **characterized in that** the housing (1) and the valve element (2) are made of polyethylene or polypropylene.

5. The device according to claim 1, 2, 3 or 4, **characterized in that** the spring element (3) is made of polyethylene or polypropylene or spring steel.

6. The device according to claim 1, 2, 3, 4 or 5, **characterized in that** the filter is made of foamed polyurethane.

## Revendications

1. Dispositif destiné à être placé dans une prothèse de trachéostome ou un plâtre de trachéostome contenant un corps cylindrique (1) et un élément à clapet cylindrique (2) et
• le corps (1) possédant un premier orifice (17) destiné à être relié au stomate sur le patient, et au moins un second orifice (14) sur le côté opposé du filtre et l'élément de soupape étant prévu au second orifice (14) du corps (1) pour fermer ledit second orifice, ledit élément à clapet étant conçu pour être fermé manuellement au moyen d'un doigt et pour être ouvert en étant rappelé élastiquement,
• le corps (1) enfermant l'élément à clapet (2) et l'élément à clapet (2) incluant un filtre à échanger l'humidité et la chaleur et un élément à ressort cylindrique (3) étant disposé entre le corps (1) et l'élément à clapet (2),
• le corps (1) possédant un anneau de guidage supérieur (11) et un anneau de guidage inférieur (12), entre lesquels se trouvent des nervures de guidage (13) en paires avec des évidements intermédiaires (131), et des orifices latérales (134) étant formés entre les nervures de guidage (13) en paires,
• l'élément à clapet (2) possédant un couvercle (23) ainsi qu'un paroi (24) avec des évidements latéraux (221), lesdits évidements (221) correspondant en position et forme aux orifices latérales (134) du corps (1), et au moins deux tenons de guidage (22) étant prévu au paroi (24) sous le couvercle (23), les tensons de guidage s'engrenant dans les évidements (131) des nervures de guidage (13) du corps (1) et
• le filtre étant positionné entre le couvercle (23) et le paroi (24) de l'élément à clapet (2) et l'élément à ressort (3) étant disposé au-dessous du filtre entre le corps (1) et l'élément à clapet (2),
est **caractérisé en ce que** l'élément à ressort (3) se compose d'un anneau élastique supérieur (31) et d'un anneau élastique inférieur (32), lesdits anneaux élastiques (31 et 32) étant reliés par des bras de ressort élastiques (33).

2. Dispositif suivant la revendication 1 est **caractérisé en ce que** le corps (1) possède des bras radiaux (15) sur le côté opposé des anneaux de guidage (11; 12).

3. Dispositif suivant la revendication 1 est **caractérisé en ce que** le bord inférieur du paroi (24) de l'élément à clapet (2) et les bras radiaux (15) du corps (1) maintiennent les anneaux élastiques (31 et 32) et les tenons de guidage (22) peuvent être déplacés entre deux fins de course dans les évidements (131) des nervures de guidage (13).

4. Dispositif suivant les revendications 1, 2 ou 3 est **caractérisé en ce que** le corps (1) et l'élément à clapet (2) se composent de polyéthylène ou polypropylène.

5. Dispositif suivant les revendications 1, 2, 3 ou 4 est **caractérisé en ce que** l'élément à ressort (3) se compose de polyéthylène ou polypropylène ou d'acier à ressort.

6. Dispositif suivant les revendications 1, 2, 3, 4 ou 5 est **caractérisé en ce que** le filtre se compose de polyuréthane en mousse.
